# EUROPEAN PATENT SPECIFICATION

(11) **EP 2 203 207 B1**
(45) Date of publication and mention of the grant of the patent: **22.08.2012**
(21) Application number: 08845372.5
(22) Date of filing: 30.10.2008
(51) Int. Cl.: A61M 16/04

(54) **Laryngeal mask with tape tab**
Kehlkopfmaske mit Verschlussbandlasche
Masque laryngienne avec languette d'attache

(30) Priority: 30.10.2007 GB 0721300
(43) Date of publication of application: 07.07.2010
(73) Proprietor: The Laryngeal Mask Company Ltd, Mahe (SC)
(72) Inventor: BRAIN, Archibald, Ian, Jeremy, Mahe (SC)
(74) Representative: Jacob, Reuben Ellis
(86) International application number: PCT/GB2008/003674
(87) International publication number: WO 2009/056834

(56) References cited:
- WO-A-2006/125986
- US-A- 4 223 671
- US-A- 4 326 515
- US-A- 5 437 273
- US-A1- 2005 051 173

## Description

The present invention relates to a laryngeal mask device and more particularly to a laryngeal mask airway device having a tab disposed near the proximal end for facilitating position control of the device and more specifically to such a device in which the tab is adapted to be biasable towards the philtrum of the patient in use.

The laryngeal mask airway device is well known and useful for establishing airways in or administering and monitoring anaesthesia in unconscious patients. The Laryngeal Mask Company Ltd. has marketed one popular laryngeal mask airway device commercially for many years as the "Classic". Such devices are described for example in U.S. Patent No. 4,509,514 and generally comprise a mask portion and an airway tube which, when in the fully inserted configuration, establishes an airway in a patient. For convenience of exposition, the term "fully inserted configuration" shall be used herein to refer to a laryngeal mask airway device that has been inserted into a patient and has the following characteristics: (1) the distal end of the mask portion is pressed against the patient's normally closed esophageal sphincter; (2) the cuff forms a seal around the patient's glottic opening; and (3) the airway tube extends from a proximal end located outside the patient's mouth to a distal portion that is coupled to the mask portion, the tube extending through the patient's mouth and the patient's natural upper airway so that the device provides a sealed airway extending from the tube's proximal end to the patient's lungs.

Although such devices have worked well, problems have been encountered during their use. In order for an effective airway to be created in the patient, a seal around the laryngeal inlet must be maintained, requiring that the device should remain stably in place during the medical procedure being performed on the patient. However, this has proven difficult to achieve with a standard LAMA device; involuntary movement of the patient during anaesthesia can cause shifting or dislodging of the LMA, reducing its effectiveness.

This can lead to loss of the airway in the patient; a potentially life threatening situation.. The medical staff may become distracted from the procedure being carried out on the anaesthetized patient if the airway is lost, because of the need to adjust the device to restore the airway. This is time-consuming, distracting and complicated for medical staff and dangerous for the patient. There is therefore a need for improved LMA, devices that remain stably in place during anaesthesis. A prior art device is disclosed in US-2005/005 11 73

It is the object of the present invention to provide a device that seeks to mitigate one or more of the above-mentioned disadvantages.

According to the invention there is provided a laryngeal mask airway device to facilitate lung ventilation in a patient, comprising an airway tube opening at one end into the interior of a hollow mask portion, the mask portion including a backplate and a cuff, the mask portion shaped to conform to and to fit readily into the actual and potential space behind the larynx and to seal around the circumference of the laryngeal inlet without penetrating into the interior of the larynx, the airway tube extending from a proximal end to a distal end and having a tab disposed near said proximal end, a sealed airway passage extending from the proximal end of the tube to the glottic opening when the cuff is at the inserted location, characterized in that the tab is adapted to be biasable towards the philtrum of the patient in use, characterized in that means for biasing the tab towards the philtrum of the patient are integrally formed with the tab.

Forming the means for biasing the tab towards the philtrum of the patient integrally with the tab provides a manufacturing advantage because the manufacture of a single piece is simpler than the manufacture of two pieces, with a subsequent attachment step.

Any type of means for biasing the tab towards the philtrum of the patient may then be selected, for example, on the basis of the correct size for a particular patient group.

A specifically designed position at which the means for biasing the tab can be positioned, integrally during manufacture, may be provided.

Being the biasing means fixed on the tab there is no risk of the biasing means slipping or moving out of place.

The biasing means is fastened to the face or head of the patient in use. This ensures that if the patient should make any involuntary movements during the medical procedure, the mask will remain more stably in place than if the biasing means were attached to an object separate from the patient.

Preferably, the means for biasing the tab towards the philtrum of the patient is adjustable. This feature ensures that the LMA device may be securely positioned in a variety of differently sized and shaped patients.

Preferably, the means for biasing the tab towards the philtrum of the patient comprises a continuous elasticated length. This feature ensures that the LMA device may be securely and conveniently positioned in a variety of differently sized and shaped patients without requiring adjustments; this would be of particular use where fast establishment of an airway is a priority.

Preferably, the means for biasing the tab towards the philtrum of the patient is fastened in use by attaching the ends of at least one length oftape to each other around the face or head of the patient. This feature ensures that the LMA device may be securely positioned in a variety of differently sized and shaped patients.

Preferably, the means for biasing the tab towards the philtrum of the patient is fastened in use by attaching the ends of the tape to each other using a hook, buckle, Velcro or knot.

Preferably, the means for biasing the tab towards the philtrum of the patient includes adhesive. This feature may assist in improving the strength of the fastening method, or if used alone, may provide an especially fast method of biasing the tab, which may be useful in emergency situations.

The tab extends outwardly in use from the airway tube in a direction extending from the chin of the patient towards the nose of the patient. This position for the tab is advantageous because when it is urged towards the philtrum, the airway seal of the mask portion over the pharyngeal inlet is increased.

Preferably, the tab is biased in use towards the philtrum of the patient in a direction perpendicular to a line extending from the nose of the patient to the chin of the patient. This increases the airway seal of the mask portion over the pharyngeal inlet.

Preferably, the tab extends from the airway tube for at least fifteen millimetres. This length provides an ideal surface for the adaptations required to bias the tab towards the philtrum.

Preferably, the tab is substantially rigid. This ensures that the tab can be used to more securely fasten the LMA device than if the tab were flexible. In the latter case, movement of the tab would cause movements of the tab relative to the patient, reducing the effectiveness of the seal over the glottic opening.

Preferably, the tab includes a first portion and a second portion, the first portion of the tab extending outwardly from the airway tube in a direction extending from the chin of the patient towards the nose of the patient, the second portion extending from the first portion at an angle with respect to the first portion, the angle being different than one hundred-eighty degrees. The angle of less than one hundred-eighty degrees reduces the extent to which the means for biasing the tab towards the philtrum will buckle when used.

Preferably, the airway tube includes a connector portion and a second portion, the connector portion including a proximal portion, a distal portion, and a flange, the flange defining the tab and being disposed between the proximal and distal portions, the distal portion being inserted into a proximal end of the second portion, the proximal portion being cylindrical. The tab therefore protrudes at an appropriate position from the tube so that it is positioned conveniently near to the patient's philtrum. If the tab were positioned further from the philtrum, the means for biasing could be less secure, reducing the effectiveness of the seal over the glottic opening.

Preferably, the tab is slidable mounted on the connector portion of the airway tube. This feature allows adjustment of the position of the tab where necessary, for example if the patient's philtrum is far from the tab once the LMA device is in fully inserted configuration.

Preferably, the tab has an inner diameter in contact with the airway tube or the connector portion, the inner diameter having a top and a bottom portion, the top and bottom portions having a ridged surface.

Preferably, the ridges are adapted to press into the connector portion when the tab is biased towards the philtrum of the patient. This feature ensures that when the tab is used for the purpose of biasing the distal end of the device against the oesophageal sphincter, it is locked into position and will not slide up and down the airway tube or connector portion.

Preferably, the distal end of the device is biased against an esophageal sphincter of the patient in use by the tape means. This stably enhances the seal over the laryngeal inlet.

Preferably, the tab is part of a connector. Optionally, the connector is removable.

An embodiment of the invention will now be described by way of nonlimiting example with reference to the accompanying figures, in which:
Figure 1A shows a side view of the LMA device
Figure 1B shows a side view of a connector portion;
Figure 1C shows a side view of an LMA device not constructed according to the invention in fully inserted configuration indicating 'folding' or 'kinking' of the biasing means;
Figure 2 shows a laryngeal mask airway device of the present invention in fully inserted confirmation;
Figure 3A shows a plan view of a tab not in accordance with the invention that is adapted to receive means for biasing the tab towards the Philtrum;
Figure 3B shows is plan view of a tab that has biasing means integrally formed therein;
Figure 4A shows a tab not in accordance with the invention in which the biasing means is slidably attached through slits in the tab;
Figure 4B shows plan view of a tab with two lengths of biasing means fixedly attached;
Figure 4C shows an embodiment not in accordance with the invention in which a single continuous length of biasing means is fixedly attached to the tab;
Figure 5 is a perspective view of a tab, indicating the ridges on the inner diameter;
Figure 6 is a perspective view of a tab on the airway tube of the device;
Figures 7 is a side view of a tab, indicating the grip of the ridges on the airway tube as the tab is biased towards the philtrum of the patient in use; and

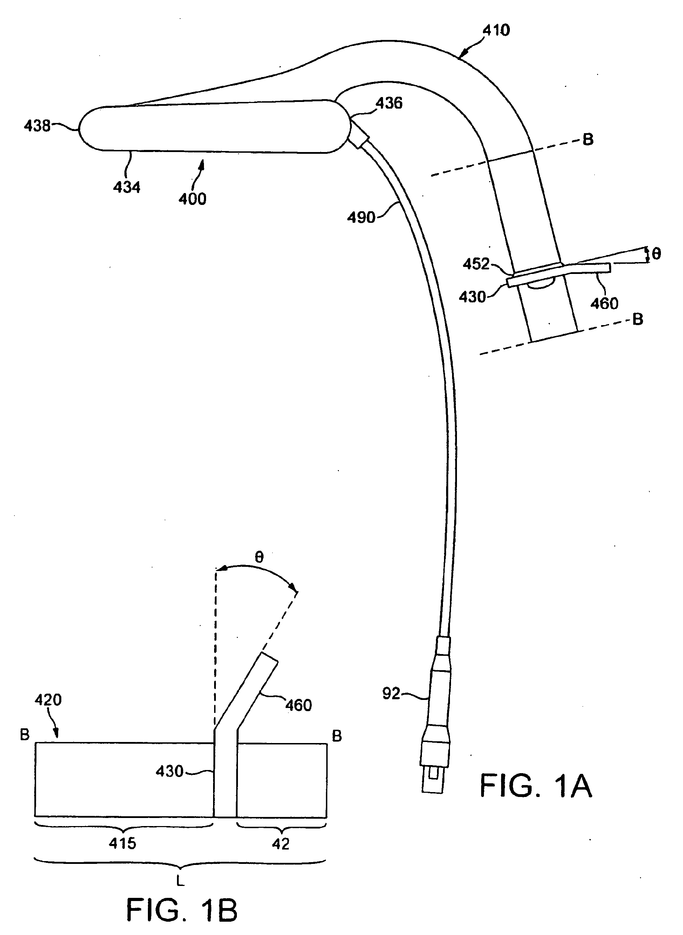

Figure 1A shows a device 400 made according to the present invention, which addresses the problems encountered during use of the prior art devices by including a tab 460, which is adapted to be biasable towards the philtrum of the patient. Device 400 includes an airway tube 410 and a mask portion 430. Mask portion includes a flat plate 415 and a cuff 434. Mask portion 430 extends from a proximal end 420 to a distal end 438. Mask portion 430 is attached to a distal portion 412 of airway tube 410. The cuff 434 may optionally be inflatable and where the cuff is inflatable the device 400 may include an inflation line 490 and a check valve 492 for use in selectively inflating and deflating the cuff 434. A position tab 460 is adapted to be biasable towards the philtrum of the patient and is disposed near the proximal end of the airway tube 410.

Figure 1B illustrates connector portion 420, which includes a proximal portion 412, a distal portion 415, and a flange 430 located between the proximal and distal portions 412, 420. Tab 460 is formed as an integral part of flange 430. Proximal portion 412 is cylindrical and is configured to couple to standard medical ventilating, or anaesthetic devices. Distal portion 420 is oblong and is configured for telescopic insertion into a proximal end 452 of integral tube and backplate portion 450. Airway tube 410 is assembled by telescopically inserting distal portion 420 into the proximal end 452 of integral tube and backplate portion 450 until flange 430 contacts proximal end 452 as shown in Figure 1A. Connector portion 420 is made of a rigid plastic or polycarbonate material. Connector portion 420 can be made, for example, by injection molding. Connector portion 420 is preferably a single monolithic piece that defines proximal portion 412, distal portion 420, flange 430, and tab 460. Flange 430 and tab 460 are preferably rigid, and are preferably rigidly fixed relative to the rest of connector portion 420. Integral tube and backplate portion 450 is also made of a plastic material such as PVC and is softer than connector portion 420. Integral tube and backplate portion 450 is characterized by a durometer of about 90 on the Shore A scale of hardness. Integral tube and backplate portion 450 may also be made by injection molding and is preferably a single monolithic piece.

Referring to Figure 1B, tab 460 extends from flange 430 at an angle theta (θ). One choice for the angle theta is fifteen degrees, which has been found to be advantageous in ensuring the maximum sealing engagement with the laryngeal inlet. Tab 460 extends from flange 430 by a height H. One choice for the height H of tab 460 is about fifteen millimeters. Flange 430 extends from a proximal portion of the airway tube in directions substantially perpendicular to the line L. In particular, flange 430 extends from a proximal portion of the airway tube in a direction substantially perpendicular to line L for a distance D, and then angles off, by the angle theta to define tab 460. One choice for the distance D is five millimetres, which has been found to be advantageous in ensuring the maximum sealing engagement with the laryngeal inlet.

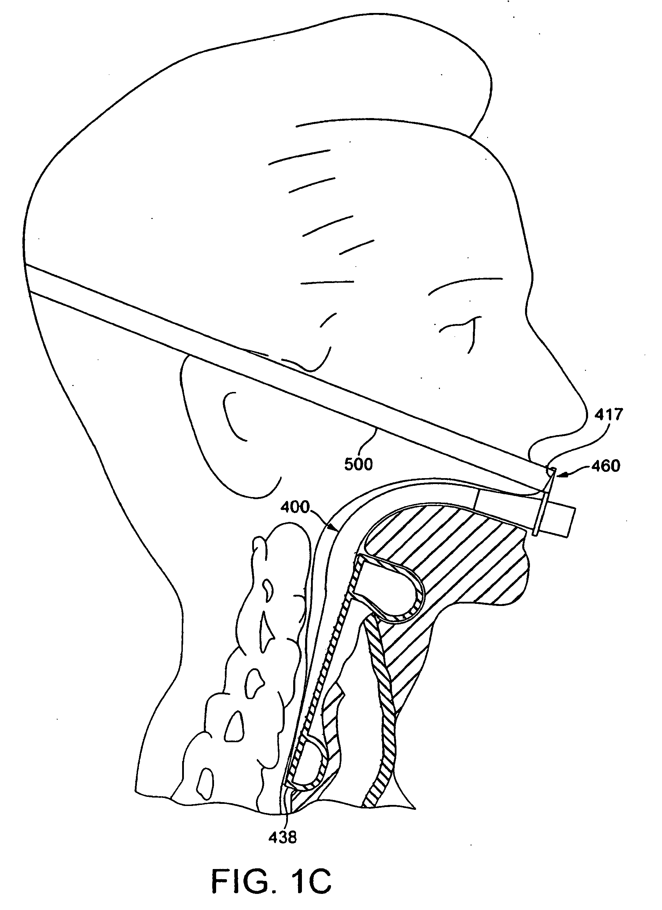

The angle of the tab is also important to avoid buckling or folding of the biasing means in use. For example, as shown in Figure 1C, if the tab 460 extends upwardly at an angle of 180°C from the airway tube in use, in a direction running from the patient's chin to the patient's nose, then the biasing means 500 may 'fold', or 'kink', if it is necessary to fix them at a particular location on the face, or around the back of the head. A 'fold' or 'kink' 417 in the biasing means, which are so important for ensuring a secure airway in the patient, could reduce the effectiveness of the airway, by introducing some 'slack'. In other words, the fold in the biasing means could become 'straightened' when the patient moves. This would increase the likelihood that the airway device would shift in the patient, reducing the seal over The laryngeal inlet. One way of addressing this issue is to design the angle of the tab so that the biasing means can be attached to the patient's face or head in a flat, unfolded, state, thus maintaining a maximally secured airway.

Another way to describe the orientation of tab 460 with respect to the airway tube is that the tab extends from the wall of the tube, which defines the tube's internal airway passage, outwardly, or away from the internal passage. When the device 400 is in the fully inserted configuration, the tab extends from the tube wall outwardly towards the patient's nose. More generally, if an up-down direction is defined as being along a line extending between the patient's nose and the patient's chin, the tab 460 extends generally in the up-down direction when the device is in the fully inserted configuration.

In addition to facilitating holding device 400 stably in the fully inserted configuration, tab 460 also facilitates insertion of device 400 into a patient and also facilitates general manipulation of the device. The proximal end of the airway tube is typically grasped and manipulated as a laryngeal mask airway device is inserted into a patient. Lubricant is typically applied to facilitate passing the mask portion through the patient's natural airway. However, the lubricant can also make the proximal end of the airway tube slippery and difficult to handle. Tab 460, which extends outwardly from the proximal end of the airway tube, provides an additional surface that may conveniently be grasped during insertion and manipulation of the device. Tab 460 thereby generally facilitates insertion and manipulation of device 400.

As discussed above, device 400 has a single tab 460 that projects generally along the patient's philtrum when the device is in the fully inserted configuration. One reason this configuration is convenient is that the patient's upper lip and cheeks are generally immobile with respect to the rest of the patient's head. In contrast, the patient's lower lip and jaw are easily moved with respect to the head and accordingly provide a less stable platform for anchoring the device 400. However, although a single tab projecting along the upper lip is a convenient configuration, it will be appreciated that other configurations of tabs may be used. For example, devices constructed according to the invention can instead include a tab that projects downward along the lower patient's lower lip, or in some other direction. Alternatively, devices constructed according to the invention can include two tabs, one projecting along the upper lip and another projecting along the lower lip, when the device is in the fully inserted configuration, and the biasing means may be integrally formed or attached to either or both of the tabs and fastened to the patient's cheeks or to other parts of the patient's head.

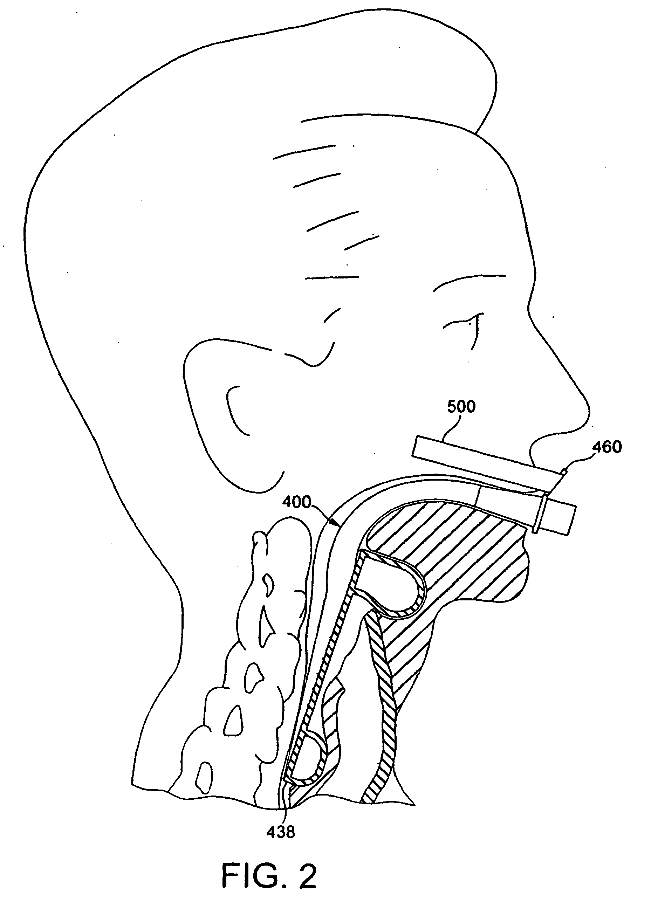

In use, the mask portion 430 is inserted through the patient's mouth into the patient's pharynx (Figure 2). The device is preferably positioned so that distal end 438 of mask portion 430 rests against the patient's normally closed esophagus and so that the end 438 of mask portion 430 is aligned with the entryway of the patient's trachea (i.e., the patient's glottic opening). After the mask portion is so positioned, the cuff forms a seal around the patient's glottic opening 450 and thus establishes a sealed airway extending from a proximal end F of airway tube 410 to the patient's trachea D. When the airway device 400 is in the fully inserted position the position tab 460 is disposed near the patient's philtrum.

Airway tube 410 translates the force from tab 460 to the distal end of the device. The force applied by incorporated tape means 600 acts to generally pull device 400 into the patient and, in particular, to simultaneously bias (a) the tab towards the patient's mouth and (b) the distal end of the device towards a more sealing engagement with the laryngeal inlet. Biasing the distal end of the device in the direction of the arrow D advantageously insures that the distal end 438 of cuff 434 remains generally in firm contact with the patient's normally closed esophageal sphincter. Ensuring that the distal end of device 300 remains in firm contact with the patient's esophageal sphincter advantageously reduces the likelihood of regurgitated material being aspirated into the patient's lungs during anaesthesia. The device can be secured using the biasing means integrally attached to the tab more quickly, conveniently and safely than the prior art devices that are fixed in place using separate means such as tape.

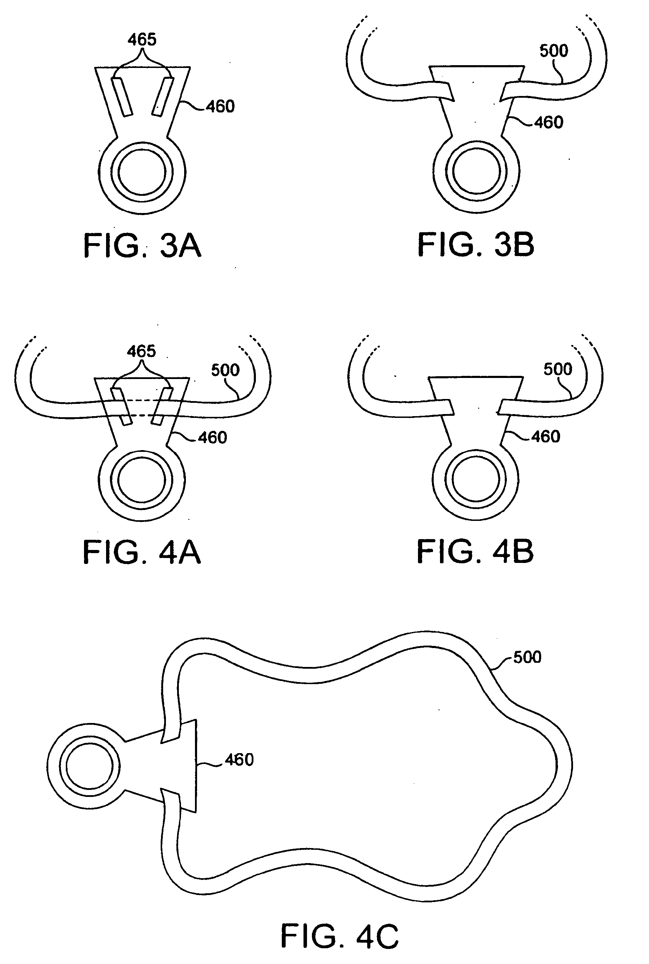

In order to bias the device 400 and increase the sealing engagement with the laryngeal inlet, the tab 460 may be adapted so that it is biased in use towards the philtrum of the patient. The tab 460 has integral means for biasing the philtrum of the patient. In other words, the biasing means 500 is integrally formed as one piece with the tab 460, for example during manufacture by moulding (Figure 3B).

The adaptation of the tab to allow it to be biased in use towards the philtrum via biasing means integrally formed with the tab has numerous advantages over the prior art. The clinician need not be concerned with the inconvenient coordination of lengths of tape, a position tab, and a patient. He can therefore fix the laryngeal mask airway device in its fully inserted configuration quickly and conveniently. This is a potentially life-saving advantage because the clinician can focus on the unconscious patient instead of on the cumbersome process of fixing lengths of tape to the tab and then to the face of the patient. The adjustable nature of the biasing means ensure that the tab can be used to bias the airway device as required in a patient having a head of any size or shape. A further advantage is that because the biasing means is integrally formed with the tab, there is no chance of the tape slipping and releasing it from its secure inserted position.

Where the patient's head cannot be rotated, it would be difficult to secure the tape by tying around the back of the head. If the tape were fixedly attached to the tab, this could present a problem in securing the airway device using the tape. Figures 4A shows a plan view of the tab 460 with a length of biasing means 500 attached to the tab 460 in a manner allowing the tape to slide through the attachment means 465 on the tab 460. The ends of the tape may therefore be tied or fastened conveniently at any location around the head or ears of the patient. Figure 4B shows an alternative embodiment in which the tab incorporates two lengths of biasing means 500 fixedly attached to the tab 460 that can be tied around the head or ears of the patient. The lengths of tape are sufficiently long that a knot or fastening can be made at any location around the head or ears of the patient. A possible variation on this embodiment could be to employ a single length of tape attached to the tab at the middle point of the length of tape. Figure 4C shows the tab 460 with one continuous length of elasticated biasing means 500 that can be stretched in use around the ears or head of the patient. The use of elasticated tape ensures that the tape is conveniently stretchable to fit any patient and would be particularly useful when the clinician must act so quickly that there is insufficient time to fasten, secure or tie two ends of the biasing means. The biasing means may also be secured with an adjustable fastening. In use, the biasing means may be secured around the patient's head or ears and adjusted using the fastening to ensure a secure fit for a patient with any size or shape of head.

The embodiments of figs. 3A, 4A, 4B and 4C do not form part of the invention.

A possible variation on any of the above embodiments is the use, additionally or alternatively, of adhesive tape so that the tape means can be attached directly to the face or head of the patient. By fastening the tape to the face or head of the patient, using adhesive, the need to fasten the tape using buckles, hooks, tying or other means is circumvented. This embodiment would be most useful in situations where the movement of the head, which would be required in order to fasten the tape around the ears or head of the patient, would be clinically inappropriate. This could also be used to enhance the strength with which the airway device is kept in place during the anaesthetic procedure, when the abovementioned methods of fixing such as tying or adjustable fastenings are used.

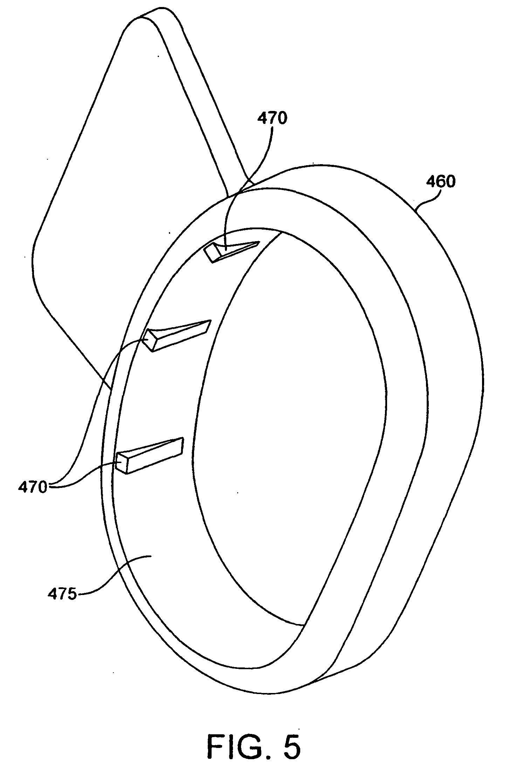

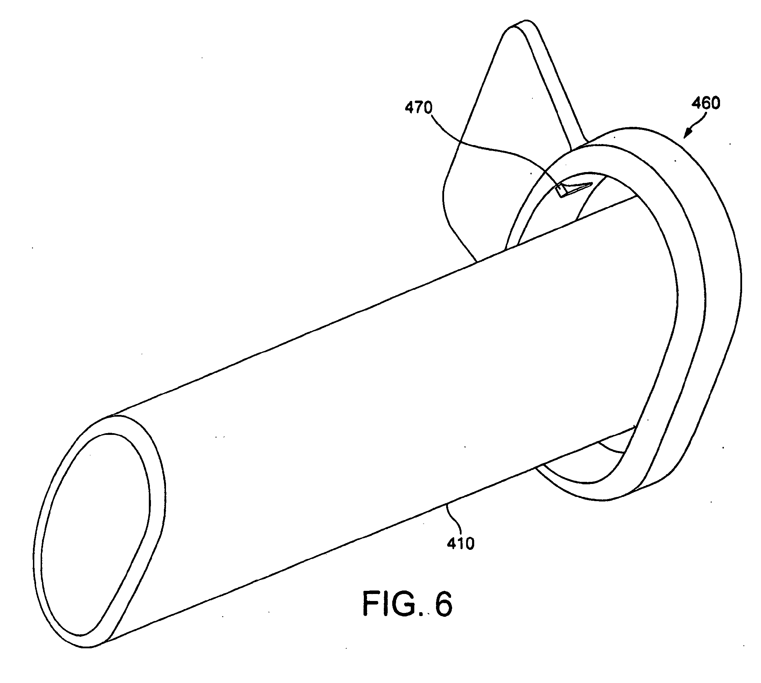

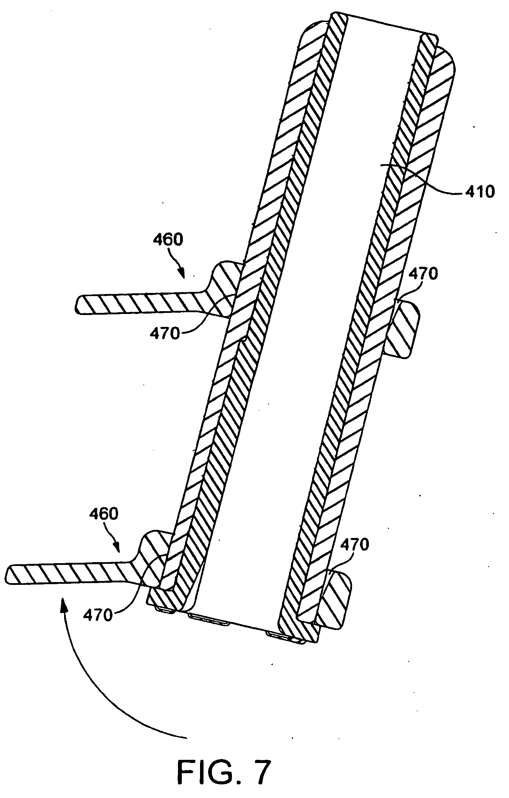

Some variation in the shape of patient's faces is observed, in particular how deep-set or prominent the philtrum is. This can create problems when the tab is biased towards the philtrum in order to stably secure the airway device in place. For example, if the tab is not biased sufficiently close to the philtrum, the length of biasing means required to reach the patient's face from the tab may be longer, creating more potential for the device to shift. The tab may therefore be adjustably positioned on the airway tube, so that it can be slid towards or away from the patient's Philtrum once the device is in fully inserted configuration. As shown in Figure 5, the inner diameter 475 of one embodiment of the tab 460, which may be in contact with the airway tube 410, may have ridges 470. Where an alternative embodiment of the invention is used, the inner diameter 475 of the tab 460 may be in contact with a connector portion 420 as described hereinabove. When the tab 460 is not being urged towards the philtrum of the patient, the tab 460 is loose and may slide up and down the airway tube 410 as shown in Figure 6. In other words, the ridges 470 are not in contact with the outer diameter of the airway tube 410 or the connector portion 420. However, when the tab 460 is biased towards the philtrum of the patient, these ridges 470 dig into the airway tube 410 or the connector portion 420 as shown in Figures 7 and 8. When the ridges 470 dig into the airway tube 410 or connector portion 420, a 'gripping' action is created that prevents the tab from slipping up or down along the airway tube 410 or connector portion 420. The tab 460 may be positioned on the connector portion 420 of the airway tube 410. In such a case, the connector portion 420 may have a 'bite block' made of softer material, into which the ridges 470 may grip with even more strength to prevent the tab 460 from slipping.

## Claims

1. A laryngeal mask airway device (400) to facilitate lung ventilation in a patient, comprising an airway tube (410) opening at one end into the interior of a hollow mask portion (430), the mask portion including a backplate (415) and a cuff (434), the mask portion shaped to conform to and to fit readily into the actual and potential space behind the larynx and to seal around the circumference of the laryngeal inlet without penetrating into the interior of the larynx, the airway tube extending from a proximal end to a distal end and having a tab (460) disposed near said proximal end, a sealed airway passage extending from the proximal end of the tube (410) to the glottic opening when the cuff (434) is at the inserted location, the tab (460) being biasable towards the philtrum of the patient in use, **characterized in that** means (600) for biasing the tab (460) towards the philtrum of the patient are integrally formed with the tab (460).

2. A device according to claim 1, wherein the means (600) for biasing the tab (460) towards the philtrum of the patient is adapted for fastening to the face or head of the patient in use.

3. A device according to any preceding claim, wherein the means (600) for biasing the tab (460) towards the philtrum of the patient is adjustable.

4. A device according to any preceding claim, wherein the means (600) for biasing the tab (460) towards the philtrum of the patient comprises a continuous elasticated length.

5. A device according to any preceding claim, wherein the means (600) for biasing the tab (460) towards the philtrum of the patient is at least one length of tape extending in use around the head of the patient, the free ends of said at least one length of tape being attached to each other.

6. A device according to claim 5, wherein the free ends of the at least one length of tape are attached to each other using a book, buckle, Velcro or knot.

7. A device according to any preceding claim, wherein the means 600 for biasing the tab (460) towards the philtrum of the patient is elasticated.

8. A device according to any preceding claim, wherein the means (600) for biasing the tab (460) towards the philtrum of the patient in use to the face or head of the patient includes adhesive.

9. A device according to any preceding claim, the tab (460) extending outwardly in use from the airway tube (410) in a direction extending from the chin of the patient towards the nose of the patient.

10. A device according to any preceding claim, the tab (460) extending from the airway tube (410) for at least fifteen millimeters.

11. A device according to any preceding claim, the tab (460) including a first portion and a second portion, the first portion of the tab (460) extending outwardly from tie airway tube (410) in a direction extending from the chin of the patient towards the nose of the patient, the second portion extending from the first portion at an angle with respect to the first portion, the angle being different than one hundred-eighty degrees.

12. A device according to any preceding claim, the airway tube (410) including a connector portion (420) and a second portion, the connector portion (420) including a proximal portion (412), a distal portion (420), and a flange (430), the flange (430) defining the tab (460) and being disposed between the proximal (412) and distal (420) portions, the distal portion (420) being inserted into a proximal end of the second portion, the proximal portion (412) being cylindrical.

13. A device according to any preceding claim, the tab (460) being slidably mounted on the connector portion (420) of the airway tube (410).

14. A device according to any preceding claim, the tab (460) having an inner diameter in contact with the airway tube (410), the inner diameter having a top and a bottom portion, the top and bottom portions having a ridged surface.

15. A device according to claim 14, the ridges being adapted to press into the connector portion (420) when the tab (460) is biased towards the philtrum of the patient.

16. A device according to any preceding claim in which the biasing of the tab (460) towards the philtrum of the patient in use causes a distal end of the device to be biased against the esophageal sphincter of the patient.

17. A device according to any preceding claim, wherein the tab (460) is part of a connector.

18. A device according to claim 17, wherein the connector is removable.

## Patentansprüche

1. kehlkopfmasken-Atemwegsvorrichtung (400) zum Erleichtern der Lungenbeat-mung bei einem Patienten, die einen Atemwegstubus (410) umfasst, der sich an einem Ende in das Innere eines hohlen Maskenabschnitts (430) öffnet, wobei der Maskenabschnitt eine Rückplatte (415) und eine Manschette (434) einschließt, wobei der Maskenabschnitt so geformt ist, dass er sich an den tatsächlichen und möglichen Raum hinter dem Kehlkopf anpasst und leicht in denselben passt und um den Umfang des Kehlkopfeingangs abdichtet, ohne in das Innere des Kehlkopfes einzudringen, wobei sich der Atemwegstubus von einem proximalen Ende bis zu einem distalen Ende erstreckt und eine Lasche (460) hat, die nahe dem proximalen Ende angeordnet ist, wobei sich ein abgedichteter Atemwegsdurchgang von dem proximalen Ende des Tubus (410) bis zu der Glottis-Öffnung erstreckt, wenn sich die Manschette (434) an der eingesetzten Position befindet, wobei die Lasche (460) bei Anwendung zum Philtrum des Patienten hin vorgespannt werden kann, **dadurch gekennzeichnet, dass** Mittel (600) zum Vorspannen der Lasche (460) zum Philtrum des Patienten hin integral mit der Lasche (460) geformt sind.

2. Vorrichtung nach Anspruch 1, wobei die Mittel (600) zum Vorspannen der Lasche (460) zum Philtrum des Patienten hin für eine Befestigung am Gesicht oder Kopf des Patienten bei Anwendung eingerichtet sind.

3. Vorrichtung nach einem der vorhergehenden Ansprüche, wobei die Mittel (600) zum Vorspannen der Lasche (460) zum Philtrum des Patienten hin einstellbar sind.

4. Vorrichtung nach einem der vorhergehenden Ansprüche, wobei die Mittel (600) zum Vorspannen der Lasche (460) zum Philtrum des Patienten hin eine durchgehende elastische Länge umfassen.

5. Vorrichtung nach einem der vorhergehenden Ansprüche, wobei die Mittel (600) zum Vorspannen der Lasche (460) zum Philtrum des Patienten hin wenigstens eine Länge eines Bandes sind, die sich bei Anwendung um den Kopf des Patienten erstreckt, wobei die freien Enden der wenigstens einen Länge eines Bandes aneinander befestigt sind.

6. Vorrichtung nach Anspruch 5, wobei die freien Enden der wenigstens einen Länge eines Bandes unter Verwendung eines Hakens, einer Schnalle, eines Klettverschlusses oder eines Knotens aneinander befestigt sind.

7. Vorrichtung nach einem der vorhergehenden Ansprüche, wobei die Mittel (600) zum Vorspannen der Lasche (460) zum Philtrum des Patienten hin elastisch sind.

8. Vorrichtung nach einem der vorhergehenden Ansprüche, wobei die Mittel (600) zum Vorspannen der Lasche (460) zum Philtrum des Patienten hin bei Anwendung am Gesicht oder Kopf des Patienten Haftmittel einschließen.

9. Vorrichtung nach einem der vorhergehenden Ansprüche, wobei sich die Lasche (460) bei Anwendung von dem Atemwegstubus (410) aus in einer Richtung nach außen erstreckt, die sich vom Kinn des Patienten zur Nase des Patienten hin erstreckt.

10. Vorrichtung nach einem der vorhergehenden Ansprüche, wobei sich die Lasche (460) um wenigstens fünfzehn Millimeter von dem Atemwegstubus (410) aus erstreckt.

11. Vorrichtung nach einem der vorhergehenden Ansprüche, wobei die Lasche (460) einen ersten Abschnitt und einen zweiten Abschnitt einschließt, wobei sich der erste Abschnitt der Lasche (460) von dem Aternwegstubus (410) aus in einer Richtung nach außen erstreckt, die sich vom Kinn des Patienten zur Nase des Patienten hin erstreckt, wobei sich der zweite Abschnitt von dem ersten Abschnitt aus in einem winkel in Bezug auf den erste Abschnitt erstreckt, wobei der Winkel anders als einhundertachtzig Grad ist.

12. Vorrichtung nach einem der vorhergehenden Ansprüche, wobei der Atemwegstubus (410) einen Verbinderabschnitt (420) und einen zweiten Abschnitt einschließt, wobei der Verbinderabschnitt (420) einen proximalen Abschnitt (412), einen distalen Abschnitt (420) und einen Flansch (430) einschließt, wobei der Flansch (430) die Lasche (460) definiert und zwischen dem proximalen (412) und dem distalen (420) Abschnitt angeordnet ist, wobei der distale Abschnitt (420) in ein proximales Ende des zweiten Abschnitts eingesetzt ist, wobei der proximale Abschnitt (412) zylindrisch ist.

13. Vorrichtung nach einem der vorhergehenden Ansprüche, wobei die Lasche (460) verschiebbar an dem Verbinderabschnitt (420) des Atemwegstubus (410) angebracht ist.

14. Vorrichtung nach einem der vorhergehenden Ansprüche, wobei die Lasche (460) einen Innendurchmesser in Berührung mit dem Atemwegstubus (410) hat, wobei der Innendurchmesser einen oberen und einen unteren Abschnitt hat, wobei der obere und der untere Abschnitt eine gerippte Oberfläche haben.

15. Vorrichtung nach Anspruch 14, wobei die Rippen dafür eingerichtet sind, sich in den Verbinderabschnitt (420) zu drücken, wenn die Lasche (460) zum Philtrum des Patienten hin vorgespannt wird.

16. Vorrichtung nach einem der vorhergehenden AnsprüChe, wobei das Vorspannen der Lasche (460) zum Philtrum des Patienten hin bei Anwendung bewirkt, dass ein distales Ende der Vorrichtung gegen den Speiseröhren-Schließmuskel des Patienten vorgespannt wird.

17. Vorrichtung nach einem der vorhergehenden Ansprüche, wobei die Lasche (460) ein Teil eines Verbinders ist.

18. Vorrichtung nach Anspruch 17, wobei der Verbinder abnehmbar ist.

## Revendications

1. Dispositif pour voies aériennes à masque laryngé (400), pour faciliter la ventilation pulmonaire chez un patient, comprenant un tube pour voie aérienne (410) débouchant, à une extrémité, à l'intérieur d'une partie de masque (430) creuse, la partie de masque comprenant une plaque dorsale (415) et une manchette (434), la partie de masque étant façonnée pour se conformer et le monter aisément dans l'espace réel et potentiel situé derrière le larynx et pour former une étanchéité autour de la circonférence de l'entrée laryngée, sans pénétrer à l'intérieur du larynx, le tube pour voie aérienne s'étendant d'une extrémité proximale à une extrémité distale et comprenant une patte (460) disposée à proximité de ladite extrémité proximale, un passage de voie aérienne isolé de manière étanche, s'étendant de l'extrémité proximale du tube (410) à l'ouverture glottique, lorsque la manchette (434) se trouve à l'emplacement inséré, la patte (460) étant susceptible d'être sollicitée vers le philtrum du patient, en utilisation, **caractérisé en ce que** des moyens (600), pour solliciter la patte (460) vers le philtrum du patient, sont formés d'une seule pièce avec la patte (460).

2. Dispositif selon la revendication 1, dans lequel les moyens (600), pour solliciter la patte (460) vers le philtrum du patient, sont adaptés pour une fixation au visage ou à la tête du patient, en utilisation.

3. Dispositif selon l'une quelconque des revendications précédentes, dans lequel les moyens (600), pour solliciter la patte (460) vers le philtrum du patient, sont ajustables.

4. Dispositif selon l'une quelconque des revendications précédentes, dans lequel les moyens (600), pour solliciter la patte (460) vers le philtrum du patient, présentent une longueur rendue élastique continue.

5. Dispositif selon l'une quelconque des revendications précédentes, dans lequel les moyens (600), pour solliciter la patte (460) vers le philtrum du patient, sont composés d'au moins une longueur de ruban, s'étendant, en utilisation, autour de la tête du patient, les extrémités libres de ladite au moins une longueur de ruban étant attachées ensemble.

6. Dispositif selon la revendication 5, dans lequel les extrémités libres de ladite au moins une longueur de ruban sont attachées ensemble en utilisant un crochet, une boucle, du Velero ou un noeud.

7. Dispositif selon l'une quelconque des revendications précédentes, dans lequel les moyens (600), pour solliciter la patte (460) vers le philtrum du patient, sont rendus élastiques.

8. Dispositif selon l'une quelconque des revendications précédentes, dans lequel les moyens (600), pour solliciter la patte (460) vers le philtrum du patient, en utilisation, sur le visage ou la tête du patient comprennent un adhésif,

9. Dispositif selon l'une quelconque des revendications précédentes, la patte (460) s'étendant extérieurement, en utilisation, à partir du tube pour voie aérienne (410), dans une direction s'étendant du menton du patient vers le nez du patient.

10. Dispositif selon l'une quelconque des revendications précédentes, la patte (460) s'étendant à partir du tube pour voie aérienne (410) sur au moins quinze millimètres.

11. Dispositif selon l'une quelconque des revendications précédentes, la patte (460) comprenant une première partie et une deuxième partie, la première partie de la patte (460) s'étendant extérieurement à partir du tube pour voie aérienne (410), dans une direction s'étendant du menton du patient vers le nez du patient, la deuxième partie s'étendant à partir de la première partie, sous un angle par rapport à la première partie, l'angle étant différent de cent quatre-vingt degrés.

12. Dispositif selon l'une quelconque des revendications précédentes, le tube pour voie aérienne (410) comprenant une partie formant connecteur (420) et une deuxième partie, la partie formant connecteur (420) comprenant une partie proximale (412), une partie distale (420) et une bride (430), la bride (430) définissant la patte (460) et étant disposée entre les parties proximale (412) et distale (420), la partie distale (420) étant insérée dans une extrémité proximale de la deuxième partie, la partie proximale (412) étant cylindrique.

13. Dispositif selon l'une quelconque des revendications précédentes, la patte (460) étant montée à coulissement sur la partie connecteur (420) du tube pour voie aérienne (410).

14. Dispositif selon l'une quelconque des revendications précédentes, la patte (460) ayant un diamètre intérieur en contact avec le tube pour voie aérienne (410), le diamètre intérieur ayant une partie supérieure et une partie inférieure, les parties supérieure et inférieure présentant une surface nervurée.

15. Dispositif selon la revendication 14, les nervures étant adaptées pour presser dans la partie connecteur (420), lorsque la patte (460) est sollicitée vers le philtrum du patient.

16. Dispositif selon l'une quelconque des revendications précédentes, dans lequel la sollicitation de la patte (460) vers le philtrum du patient, en utilisation, provoque une sollicitation d'une extrémité distale du dispositif coutre le sphincter oesophagien du patient.

17. Dispositif selon l'une quelconque des revendications précédentes, dans lequel la patte (460) fait partie d'un connecteur.

18. Dispositif selon la revendication 17, dans lequel le connecteur est amovible.
